# EUROPEAN PATENT APPLICATION

(11) **EP 1 099 425 A1**
(43) Date of publication of application: **16.05.2001**
(21) Application number: 99308973.9
(22) Date of filing: 11.11.1999
(51) Int. Cl.: A61F 2/06

(54) **Stent, manufacturing method therof and indwelling method therof**

(30) Priority: 11.11.1999 JP 32110299
(71) Applicant: Ogawa Spring Co.,Ltd., Kasukabe-shi, Saitama-ken (JP)
(72) Inventor: Sugita, Yoichi, Suginami-ku, Tokyo (JP); Ogawa, Akira, Kasukabe-shi, Saitama-ken (JP); Kyo, Kenji, Kasukabe-shi, Saitama-ken (JP)
(74) Representative: Grünecker, Kinkeldey, Stockmair & Schwanhäusser Anwaltssozietät

(57) **Abstract**

A stent, a manufacturing method thereof and indwelling method thereof are provided, the stent being capable of increasing diameter change enlarging and contracting in accordance with temperature change for being freely selectable and applicable to different diameter of intracorporeal lacuna, thereby improving safety and reducing burden of treatment for patients. The stent has a hollow cylindrical body (3) by braiding a plurality of filament body (2) made of Ti-Ni alloy of excessive Ni. A lower-temperature shape memorizing process and a higher-temperature shape memorizing process are conducted to the hollow cylindrical body (3) to memorize first shape of lower-temperature and second shape of higher-temperature having mutually different cylindrical diameter and the cylindrical diameter changes in accordance with temperature change. The crossing filament bodies (2) are fixed only at both ends (3A, 3B) of the stent.

## Description

### BACKGROUND OF THE INVENTION

### 1.FIELD OF THE INVENTION

The present invention relates to a stent, a manufacturing method thereof and indwelling method thereof, the stent being capable of opening intracorporeal lacuna for a long time for treatment of stricture of blood-vascular system and non-blood-vascular system such as bile duct and digestive tract, and obstructive lesion, which has attracted attention in the field of so-called IVR (Interventional Radiology) treatment.

### 2.DESCRIPTION OF RELATED ART

Broadly speaking, metal stents are classified into two types: one is set around a balloon catheter to be expanded and indwelled by the balloon expansion; and the other has expansibility by itself such as Z-stent and "wall-stent". Stainless steel, shape memory alloy or the like are used as material of the stent.

In order to indwell a stent having expansibility by itself, the stent such as the Z-stent is pushed out of a catheter to be released and indwelled, or a stent is set around a catheter-shaped core and covered with a sheath and is released when the sheath portion is drawn backward.

However, since position of such stents cannot be corrected once expanded in the intracorporeal lacuna, additional stents are generally indwelled, which makes the treatment difficult, increases burden on patients and deteriorates safety of the treatment.

In view of the above, a stent using bidirectional shape memory alloy is known (Japanese Patent Publication Hei 3-51430).

The stent uses bi-directional shape memory alloy wire composed of Ni-Ti type, Cu-Al-Ni type and Cu-Zn-Al type alloy formed in a shape capable of expanding and contracting in diametral direction thereof, more specifically, in spiral shape, in spiral cylindrical shape, in cylindrically braided shape, or in pipe shape. The stent keeps a form expanded in diametral direction thereof at or around body temperature (35°C to 37°C, for example) and becomes a form contracted in diametral direction at a temperature substantially lower than the body temperature (15°C to 20°C, for example).

However, though the diameter of the stent using the bidirectional shape memory alloy expands and contracts in accordance with temperature change, the diameter change remains only small amount.

According to the description of the publication (Japanese Patent Publication Hei 3-51340), the stent having Ni-Ti type bidirectional shape memory alloy (containing approximately 51 atomic weight % of Ni) of 0.04mm thick and 1mm width has inner diameter of approximately 2.0mm at body temperature and contracts into approximately 1.4mm of inner diameter at a temperature not more than 15°C, which shows only approximately 0.6mm of difference.

Accordingly, the stent cannot be indwelled in intracorporeal lacuna and cannot be moved while firmly wound around a catheter.

Further, the stent shown in the publication is not suited for practical use.

In other words, when deformation in two directions occurs, desired deformation cannot be securely obtained unless the material is suitably selected and thermal treatment is appropriately conducted, which results in difficulty in accurately controlling the shape and generated force.

Accordingly, the above-described stent using the bidirectional shape memory alloy has not been made into practical use at present.

### SUMMARY OF THE INVENTION

To solve the above-described problems, an object of the present invention is to provide a stent and manufacturing method and indwelling method thereof, the stent capable of increasing change of diameter etc. enlarging and contracting in accordance with temperature change and capable of configured into any shape at higher temperature and lower temperature, the stent therefore being suitably selected and used to different intracorporeal lacuna diameter, so that indwelling to the intracorporeal lacuna and position correction after indwelling can be facilitated to enhance safety and reduce burden for the patient.

In order to achieve the above object, a stent according to the present invention is arranged as follows.

A stent according to the present invention has a hollow cylindrical body made of a plurality of filament body of Ti-Ni alloy having excessive Ni, the hollow cylindrical body memorizing first shape during lower-temperature shape memorizing process and second shape during higher-temperature shape memorizing process and being deformable between the first shape and the second shape in accordance with temperature change.

In other words, the stent is formed in the hollow cylindrical body by braiding the plurality of filament body, where the hollow cylindrical body is deformable into mutually different first shape and the second shape. The lower-temperature shape memorizing process is conducted in the first shape condition and the higher-temperature shape memorizing process is conducted in the second shape, so that reversible deformation is possible between the two shapes in accordance with temperature change, in which the hollow body approaches the first shape in lower temperature and approaches the second shape in higher temperature.

The deforming shape may be any shape between the first shape and the second shape. In other words, between the first shape and the second shape, reversible deformation within an area from a shape similar to the first shape and slightly close to the second shape to a shape similar to the second shape and slightly close to the first shape, may also be possible.

The Ti-Ni alloy includes at least Ti and Ni. However, other chemical element may be contained therein.

According to the present invention, since the hollow cylindrical body formed by braiding the plurality of filament body of Ni-Ti-alloy having excessive Ni experiences the lower-temperature shape memorizing process in the first shape and the higher-temperature shape memorizing process in the second shape to memorize both shapes, the hollow cylindrical body can change the shape thereof from the first shape to the second shape or from the second shape to the first shape, in accordance with temperature change.

Therefore, when the first shape and the second shape are set to have smaller diameter in lower-temperature and larger diameter in higher-temperature to secure enough difference between respective cylinder diameter, the diameter change enlarging and contracting in accordance with temperature change can be increased relative to the conventional stent and the enlarged and contracted shapes can be formed in any desired shape.

Further, the first shape and the second shape can be set in any configuration. For instance, the change in accordance with temperature may not be change in diametral dimension but may be change in axial line. For instance, the first shape may be linear tube and the second shape may be curved or bent cylindrical shape such as J-shape, L-shape. C-shape, U-shape and S-shape.

Further, the hollow cylindrical may not be consecutive cylinder having constant diameter, but either the first shape or the second shape may be a tapered tube shape having gradually changing diameter dimension. As described below, only end portion of the cylinder may be enlarged in tapered manner.

In the stent according to the present invention, the lower-temperature shape memorizing process may preferably be a solution thermal treatment and the higher-temperature shape memorizing process may preferably be a constraint aging thermal treatment.

Accordingly, the shape memorization in the first shape and the second shape can be further securely conducted, thereby accurately controlling the respective shapes and generated force for increasing change between respective shapes.

In the stent according to the present invention, the first shape preferably have smaller cylinder diameter than the second shape.

According to the above, the stent can substitute the above-described conventional stent. And since the diametral change enlarging and contracted in accordance with temperature change can be increased relative to conventional stent, the stent can be freely selected and applied to intracorporeal lacuna having different diameter, so that indwelling to the intracorporeal lacuna and correction of the indwelled position can be easily conducted, thereby improving safety to the patient and reducing burden of the treatment.

In the stent according to the present invention, crossed portion of the filament body may preferably be fixed only at both ends of the hollow cylindrical body.

Accordingly, since the crossed portion of the filament body is fixed on both ends of the hollow cylindrical body and the crossed portions of the filament body other than both ends are set free, the change in longitudinal direction can be reduced even when the diameter of the hollow cylindrical body is enlarged and contracted in accordance with temperature change. In other words, since the crossed portion of the filament body are fixed only on both ends of the hollow cylindrical body, the longitudinal change can be reduced while allowing large diameter change enlarging and contracting in accordance with temperature change, thereby reducing indwelling error in indwelling the stent into the intracorporeal lacuna as little as possible

In the stent according to the present invention, the first shape of the hollow cylindrical body may preferably have smaller diameter than the second shape and the second shape may preferably have larger diameter of both opening ends than an intermediate portion thereof.

In the conventional stents, after being inserted in the intracorporeal lacuna in small-diameter condition in lower-temperature, the stent deformed in large-diameter condition of higher-temperature. In the deformation, though desired diameter enlargement can be obtained at the intermediate portion, it is known by experience that sufficient diameter enlargement cannot be obtained on both opening ends, which may cause disadvantage of humor flow toward outer circumference of the stent and generation of thrombus etc. at that time.

On the other hand, when the both opening ends are enlarged to have larger diameter than the intermediate portion during enlarged condition, the both opening ends enlarges to the same diameter as the intermediate portion or larger, thereby preventing humor flow into the outer circumference of the stent. Further, since the end portion of the stent firmly catches wall of intracorporeal lacuna, unnecessary movement of the stent can be prevented.

In the stent according to the present invention, one or more than one of the plurality of filament body may preferably be made of gold or tantalum.

Accordingly, since the hollow cylindrical body includes the filament body of gold or tantalum photographable under fluoroscopy of roentgen rays, shadowing effect by X-rays can be expected, so that the position of the stent can be checked by roentgen rays when the stent is indwelled or withdrawn from the body. Accordingly, safe operation can be secured.

In the stent according to the present invention, the Ti-Ni alloy may preferably contain Co or Cu.

Accordingly, transformation temperature and deformation rate can be changed by the added elements, thereby being capable of selectively producing expansive soft stent or expansive rigid stent in accordance with usage.

Manufacturing method of the stent according to the present invention includes following arrangement.

A manufacturing method of a stent according to the present invention includes the steps of: braiding a plurality of filament body of Ti-Ni alloy of excessive Ni to form a hollow cylindrical body deformable into mutually different first shape and second shape; memorizing lower-temperature shape for memorizing the first shape to the hollow cylindrical body as a lower-temperature shape; and memorizing higher-temperature shape for memorizing the second shape to the hollow cylindrical body as a higher-temperature shape.

Accordingly, the first shape as the lower-temperature shape can be memorized to the hollow cylindrical body during the lower-temperature shape memorizing process and the second shape as the higher-temperature shape can be memorized during he higher-temperature shape memorizing process. Therefore, the first shape and the second shape can be securely memorized, thereby increasing shape change, enlargement and contraction for example, in accordance with temperature change and changing the hollow cylindrical body into any desired shape. Accordingly, the stent can be selectively freely applied to the intracorporeal lacuna having different diameter, thereby facilitating indwelling into body and correcting indwelled position, so that safety to the patient can be improved and burden on the patients can be reduced.

In the manufacturing method of the stent according to the present invention, a solution thermal treatment may preferably be conducted during the lower-temperature shape memorizing step while keeping the hollow cylindrical body in the first shape, and an aging thermal treatment may preferably be conducted during the higher-temperature shape memorizing step while binding the hollow cylindrical body in the second shape.

According to the above arrangement, shape memorization of the first shape and the second shape can be further securely conducted, so that respective shapes and generated force can be accurately controlled and the change between the respective shapes can be further magnified.

An indwelling method of a stent according to the present invention includes the following steps.

An indwelling method of a stent according to the present invention includes the steps of: providing a stent having first shape of lower-temperature with larger cylinder diameter than cylindrical diameter of second shape of higher-temperature; accommodating the stent into a sheath after making it thinner than the cylinder diameter of the first shape; inserting a distal end of the sheath adjacent to indwelling position of intracorporeal lacuna; and inserting a rod into the sheath and pushing out the stent in the sheath to indwell the stent in the intracorporeal lacuna.

Accordingly, since the diameter change enlarging and contracting in accordance with temperature change of the stent itself is large and the stent has approximately one ninth of the elastic modulus relative to stainless steel in martensitic structure, the stent can be accommodated in a thin sheath after applying outside force to make the cylinder diameter thinner. Therefore, the treatment can be facilitated as compared to a method in which the stent is held on an outside of distal portion of the catheter to be inserted into the intracorporeal lacuna.

### BRIEF DESCRIPTION OF THE DRAWINGS

Fig. 1 is a perspective view showing an embodiment of the stent according to the present invention;
Fig. 2 is a front elevation showing the stent changed into lower temperature shape (contracted);
Fig. 3 is a front elevation showing the stent changed into higher temperature shape (expanded);
Fig. 4 is a perspective view showing constraint condition of hollow cylindrical body in lower temperature shape memorizing process of the stent;
Fig. 5 is a perspective view showing constraint condition of hollow cylindrical body in higher temperature shape memorizing process of the stent;
Fig. 6 is a graph showing diameter and length change in accordance with temperature change of the stent;
Fig. 7 is an illustration of balloon catheter used for indwelling the stent into body;
Fig. 8(A) to Fig. 8(E) are illustrations showing process for indwelling the stent using the balloon catheter;
Fig. 9(A) to Fig. 9(G) are illustrations showing process for correcting indwelling position of the stent using the balloon catheter;
Fig. 10(A) and 10(B) are illustrations showing how the stent is indwelled in the body using a sheath;
Fig. 11(A) to Fig. 11(C) are illustrations showing other example for correcting the position of the stent;
Fig. 12 is an illustration showing other embodiment of the stent according to the present invention; and
Fig. 13(A) and 13(B) are illustrations showing first and second shape of still other embodiment of the stent according to the present invention.

### DETAILED DESCRIPTION OF PREFERRED EMBODIMENT(S)

An embodiment of the present invention will be described below with reference to attached drawings.

### [Structure of Stent]

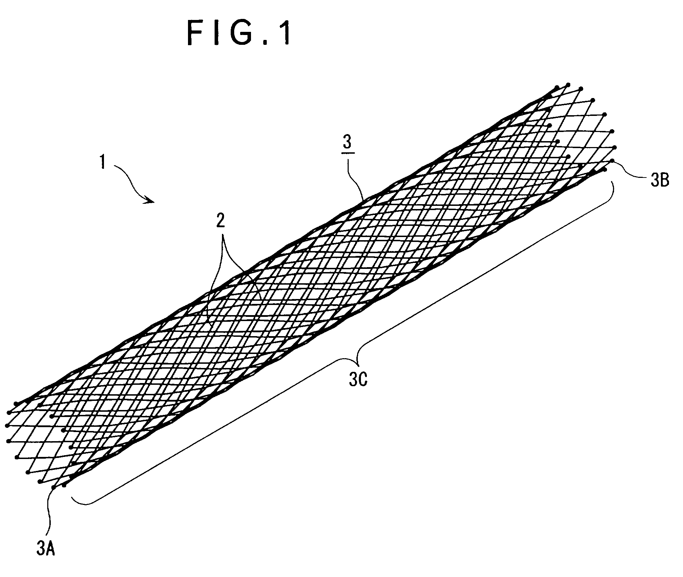

Fig. 1 is an outside perspective view of a stent 1 according to the present embodiment. The stent 1 is formed into a hollow cylindrical body 3 by braiding a plurality of filament body 2 of Ti-Ni alloy containing excessive Ni. Lower temperature shape memorizing process and higher temperature shape memorizing process are conducted to the hollow cylindrical body 3 while varying the diameter of the cylinder, so that first shape (lower temperature shape) and second shape (higher temperature shape) with different cylinder diameter are memorized and the cylinder diameter changes in accordance with temperature change.

The filament body 2 may be wire of Ti-Ni-Co alloy and Ti-Ni-Cu alloy as well as the Ti-Ni alloy or band-shaped body composed of the same. In this case, Ni content may preferably be not less than 50.5 wt%, more preferably, 54.0 to 56.5 wt%.

The thickness and number of the filament body 2 can be determined considering hardness and flexibility of the stent 1 required in accordance with indwelled portion of the stent 1. However, the number may preferably be multiple of four, specifically, eight, sixteen and thirty-two and the like. For example, using twelve Ti-Ni wires and four tantalum wires (photographable under fluoroscopy of roentgen rays), the filament body 2 may be formed into the hollow cylindrical body 3 by alternately crossing (braiding) respective filament bodies 2 up and down. In this case, by braiding the respective filament bodies 2 so that the crossing angle of the respective filament body 2 becomes around 120 degrees, pressure endurance of the stent 1 can further be enhanced.

Only the crossed portions of the filament body 2 of the hollow cylindrical body 3 are fixed at both ends, and the crossed portions of the filament body 2 other than the portion except for both ends of the hollow cylindrical body 3 are in free condition.

More specifically, the crossed portions of sixteen filament bodies 2 are fixed on both ends 3A and 3B of the hollow cylindrical body 3 by welding etc., and the crossed portion of the filament bodies 2 at an intermediate portion 3C are kept being braided (touching with each other while crossing at a predetermined crossing angle). In other words, since the filament bodies 2 are mutually touching while being capable of freely changing crossing angle therebetween at the intermediate portion 3C of the hollow cylindrical body 3 except for the both ends 3A and 3B, the diameter change in accordance with temperature change can be increased.

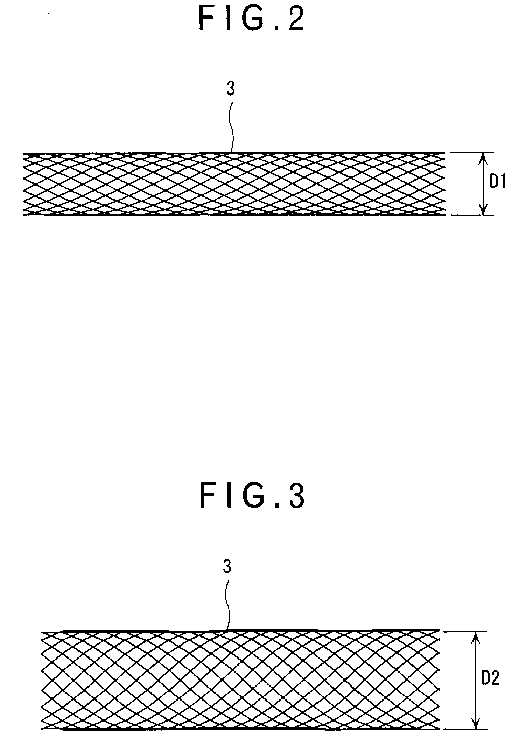

Further, the hollow cylindrical body 3 memorizes the lower-temperature shape and the higher-temperature shape having different cylindrical diameter by the lower-temperature shape memorizing process and the higher-temperature shape memorizing process while changing the cylinder diameter. In other words, in lower temperature, when the temperature of the hollow cylindrical body 3 is lower than the body temperature, for example, the hollow cylindrical body 3 is shape-memorizing processed, so that the diameter becomes first diameter D1 as shown in Fig. 2. On the contrary, in higher temperature, when the temperature of the hollow cylindrical body 3 is around the body temperature higher than the first temperature, for example, the diameter changes into second diameter D2 larger than the first diameter D1 as shown in Fig 3.

Specifically, in an example of dog abdominal aorta, when the temperature of the hollow cylindrical body 3 is around 15°C (approximately 12°C to 18°C), the diameter of the hollow cylindrical body 3 becomes approximately 5 to 8mm. When the temperature of the hollow cylindrical body 3 is approximately 34°C to 37°C, the diameter of the hollow cylindrical body 3 becomes approximately 12 to 16mm. Under an environment of temperature of 5°C to 10°C, since the hollow cylindrical body is martensitic structure and has elastic modulus of one ninth of stainless steel, which means relatively soft, the cylinder diameter can be further reduced to around 2mm when an outside force is applied to narrow the hollow cylindrical body 3. [Manufacturing Method of Stent]

The manufacturing method of the stent includes following steps.
1) Braiding step for forming the hollow cylindrical body 3 by braiding a plurality of filament body 2 made of Ti-Ni alloy having excessive Ni.
2) Fixing step for fixing crossed portions of the filament body 2 at both ends of the hollow cylindrical body 3.
3) Lower-temperature shape memorizing process for conducting solution thermal treatment on the hollow cylindrical body 3 to memorize the lower-temperature shape.
4) Higher-temperature shape memorizing process for conducting constraint aging thermal treatment while binding the hollow cylindrical body 3 in a cylinder diameter different from the lower-temperature shape to memorize the higher-temperature shape.

In the braiding step, a plurality of filament body 2 (sixteen Ti-Ni wires or twelve Ti-Ni wires and four tantalum wires) is wound around a cored bar of 20mm diameter and is braided (plaiting respective filament body 2 alternately crossing up and down) to form the hollow cylindrical body 3. Incidentally, the thickness, number and braid pitch may be appropriately selected in accordance with the intracorporeal lacuna to be applied with the stent.

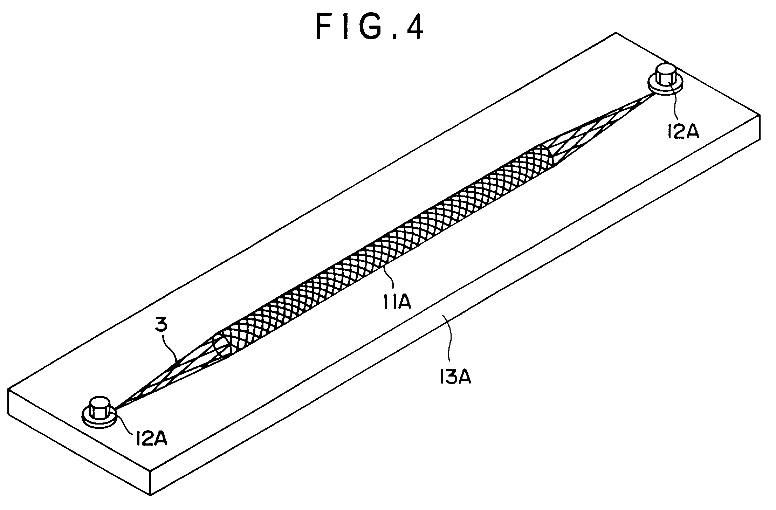

In the lower-temperature shape memorizing process, the hollow cylindrical body 3 is inserted into a cored bar (stainless steel bar) 11A having diameter of, for example, 2mm as shown in Fig. 4, the hollow cylindrical body 3 is restricted to the cored bar 11A by narrowing inner diameter thereof by stretching both ends, and the both ends are firmly fixed to a steel plate 13A with a screw 12A.

The lower-temperature shape memorizing process is conducted under the above condition. During the lower-temperature shape memorizing process, solution thermal treatment is conducted to the hollow cylindrical body 3 under Fig. 4 condition at a temperature of more than 600°C. More specifically, the hollow cylindrical body 3 is heated for approximately one hour at a temperature around 700°C to 1000°C and is cooled subsequently.

In the fixing step, the hollow cylindrical body 3 having experienced with lower-temperature shape memorizing process is inserted to a cored bar having diameter of, for example, 11mm. While both ends of the hollow cylindrical body 3 is stretched to restrict to the cored bar (i.e. enlarging larger 1 than the first diameter D1), the crossed portions of the filament body 2 are welded on the both ends.

More specifically, the hollow cylindrical body 3 is wound around an electrode of a welding machine (an electrode having a diameter of approximately 11mm), and the crossed portions of the filament body 2 on both ends are welded in this condition.

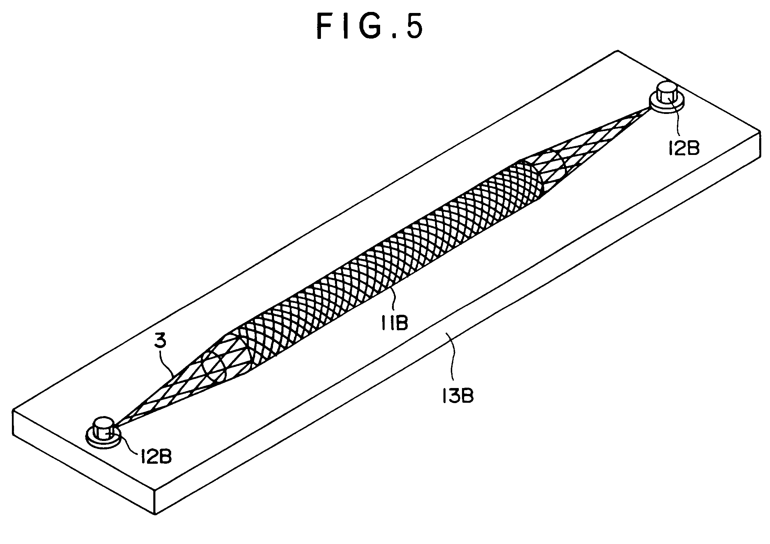

In the higher-temperature shape memorizing process, the hollow cylindrical body 3 having experienced the fixing step is inserted to a cored bar having a diameter of, for example, 15mm as shown in Fig. 5, and both ends thereof are stretched so that the hollow cylindrical body 3 is restricted to the cored bar 11B and the both ends are firmly fixed to a steel plate 13B by a screw 12B.

The higher-temperature shape memorizing process is conducted under the above condition. During the higher-temperature shape memorizing step. constraint aging thermal treatment is conducted to the hollow cylindrical body 3. More specifically, the hollow cylindrical body 3 is heated at a temperature of approximately 300°C to 560°C for about one hour and is cooled subsequently.

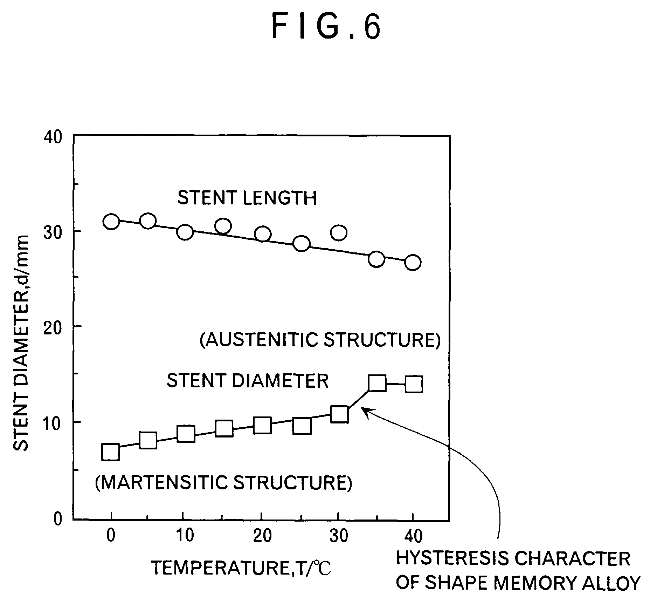

Consequently, as shown in Fig. 6, the stent 1 is obtained, in which the diameter of the hollow cylindrical body 3 is around 8mm when the temperature of the hollow cylindrical body 3 is approximately 15°C and the diameter of the hollow cylindrical body 3 changes to around 14mm when the temperature of the hollow cylindrical body 3 becomes approximately 34°C to 37°C. Further, as can be seen from Fig. 6, the length of the stent 1 changes small (the length changes approximately 4mm).

### [Using Method of Stent]

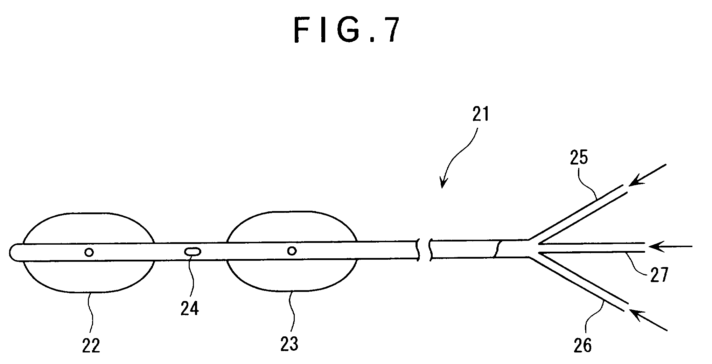

In order to indwell the stent 1 to a target position of the intracorporeal lacuna, a balloon catheter 21 shown in Fig. 7 is used.

A bloodstream shut balloon 22 and a stent moving balloon 23 are provided spaced apart at a predetermined interval at a distal portion thereof and a flush hole 24 is formed therebetween. The bloodstream shut balloon 22 and the stent moving balloon 23 are expanded by fluid supplied from branch induction tubes 25 and 26. Cold physiological saline or warm physiological saline supplied from a branch induction tube 27 is discharged from the flush hole 24.

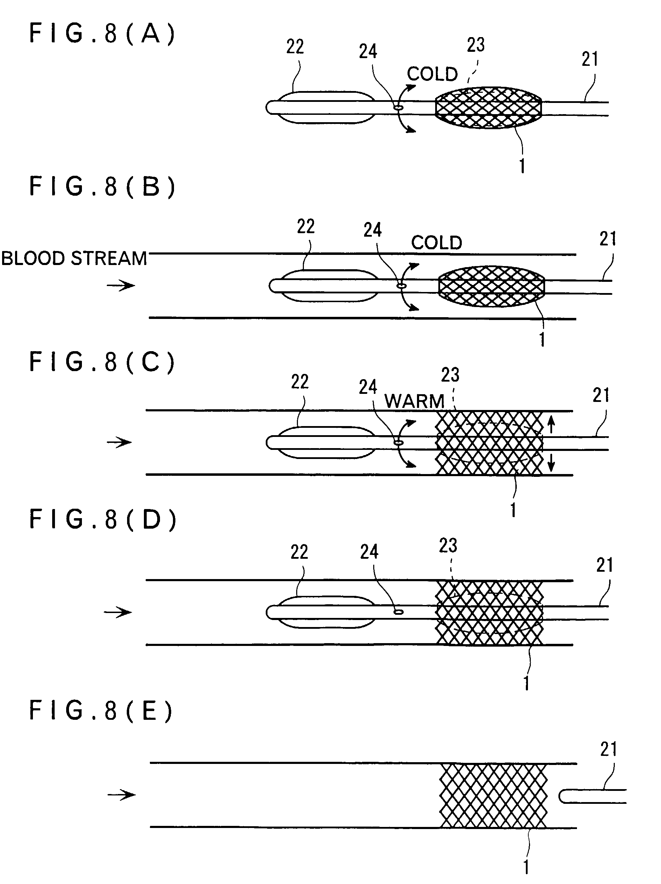

When the stent 1 is indwelled, the stent 1 is inserted to a distal position of the catheter 21 (a position corresponding to the stent moving balloon 23) as shown in Fig. 8 and the cold physiological saline is discharged from the flush hole 24 of the catheter 21. Then, the stent 1 is cooled and contracted by the cold physiological saline from the flush hole 24 and is firmly touched on the distal portion of the catheter 21 through the stent moving balloon 23 (see Fig. 8(A)).

While maintaining the above condition, the stent 1 is introduced to a target indwelling position by virtue of guide wire introduced in the intracorporeal lacuna beforehand (see Fig. 8(B)), and subsequently, the discharge of the cold physiological saline is suspended and warm physiological saline is discharged (see Fig. 8(C)). Then, the stent 1 is rapidly warmed by the warm physiological saline and is retained on an inner wall of the intracorporeal lacuna while being expanded. Under this condition, since the stent 1 is warmed by body temperature, the stent is retained under the diameter when the discharge of the warm physiological saline is suspended (see Fig. 8(D)). Thereafter, the catheter 21 is pulled out (see Fig. 8(E)).

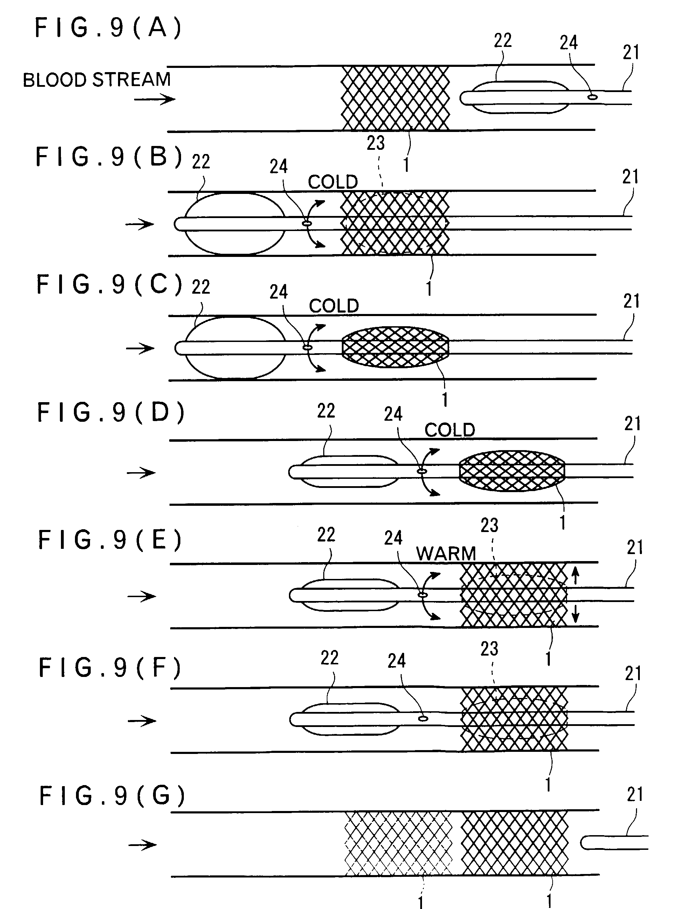

On the other hand, as shown in Fig. 9, when the stent 1 is to be withdrawn or the indwelling position thereof is to be moved, the distal portion of the catheter 21 is inserted through an inside of the stent 1 (see Fig. 9(A)) to a position where the stent moving balloon 23 corresponds to the stent 1.

At this position, the bloodstream shut balloon 22 and the stent moving balloon 23 are expanded and cold physiological saline is discharged from the flush hole 24 of the catheter 21 (see Fig. 9(B)). Then, since the stent 1 is cooled and contracted, the stent 1 is held on the distal portion of the catheter 21 through the stent moving balloon 23 (see Fig. 9(C)).

Subsequently, while the bloodstream shut balloon 22 is contracted after the stent 1 is moved to a new indwelling position (see Fig. 9(D)), the warm physiological saline is discharged from the flush hole 24 of the catheter 21 (see Fig. 9(E)). Then, the stent 1 is rapidly warmed and expanded by the warm physiological saline, the stent 1 is held on the inner wall at the indwelling position (see Fig. 9(F)). Since the stent 1 is warmed by the body temperature and the diameter is maintained when the discharge of the warm physiological saline is suspended, the catheter 21 can be pulled out from the intracorporeal lacuna (see Fig. 9(G)).

Incidentally, the scope of the present invention is not limited to the aforesaid embodiment but includes other embodiment and modifications.

In the aforesaid embodiment, the stent 1 is indwelled in the body, moved from the indwelled position and withdrawn from the body using the balloon catheter having two balloons 22 and 23 at a distal portion thereof. However, the stent 1 can be indwelled in the body by other method.

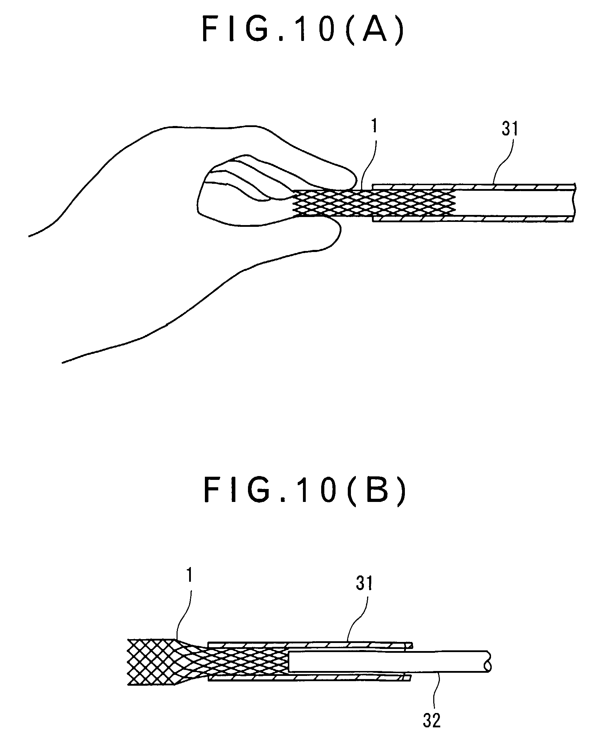

For instance, as shown in Fig. 10, the stent 1 may be picked by fingers to make the stent thinner than the cylinder diameter of the lower-temperature shape to accommodate in a sheath 31 (see Fig. 10(A)). After a distal end of the sheath 31 is inserted adjacent to indwelling position in the intracorporeal lacuna, a pushing rod 32 may be inserted into the sheath 31 to push out the stent 1 in the sheath 31 (see Fig. 10(B)) to indwell the stent 1 in the intracorporeal lacuna.

Accordingly, since the diameter itself of the stent 1 largely changes in accordance with temperature change and elastic modulus thereof is one ninth of stainless steel (meaning soft) in martensitic structure, the cylinder diameter can be further reduced by applying outside force to narrow the diameter, so that the stent 1 can be accommodated in the thin sheath 31. Accordingly, the stent 1 can be indwelled in the intracorporeal lacuna while reducing the burden on the patients, thereby more easily and safely conducting treatment than, for instance, a method for retaining the stent on a distal outside of the catheter to insert into the intracorporeal lacuna.

Further, the position of the stent 1 can be corrected according to a method other than the method described in the aforesaid embodiment.

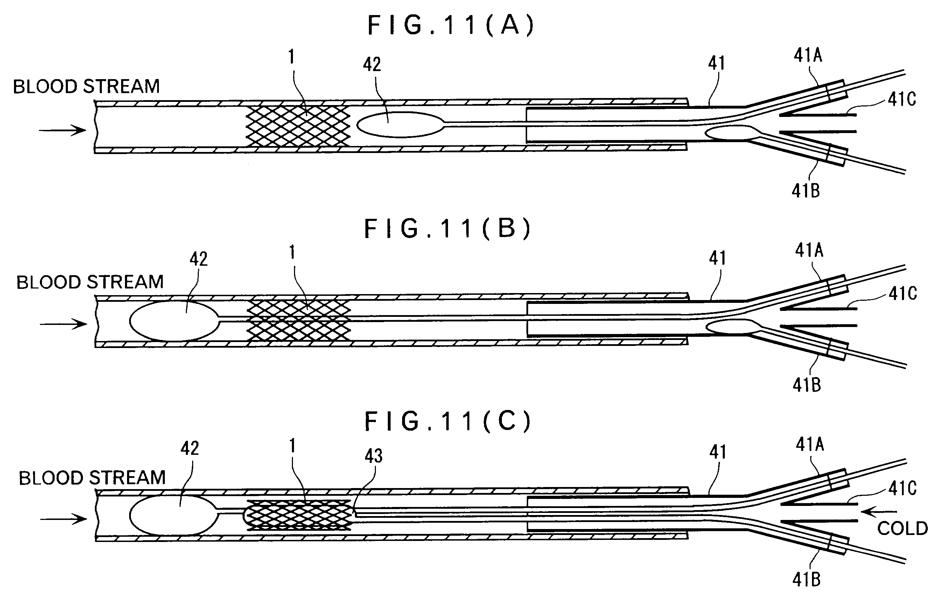

For instance, as shown in Fig. 11, after inserting a sheath 41 having a check-valve-attached balloon inserts 41A and 41B and cold physiological saline inlet 41C at a base end thereof, first balloon catheter 42 is inserted from the balloon insert 41A (see Fig. 11(A)).

After further advancing the first balloon catheter 42 to be located in front of the stent 1 relative to the bloodstream, the first balloon catheter 42 is expanded at the position (see Fig. 11(B)).

Subsequently, after inserting second balloon catheter 43 from the balloon inlet 41B to be located in the stent 1, the cold physiological saline is injected from the cold physiological saline inlet 41C. Then, the stent 1 is cooled and contracted and is touched and held by the second balloon 43 (see Fig. 11(B)). When the first balloon catheter 42 is contracted from the condition, the stent 1 becomes movable, and is capable of moving at any position.

The stent itself can be modified as follows.

Though the cylinder diameter is changed between the lower-temperature shape memorizing process and the higher-temperature shape memorizing process so that the cylinder diameter in lower-temperature shape (first shape) is larger than the cylinder diameter in higher-temperature shape (second shape), the shape memorizing process may be conducted for establishing reverse configuration. In other words, the shape memorizing process may be conducted changing the cylinder diameter between the lower-temperature shape memorizing process and the higher-temperature shape memorizing process so that the cylinder diameter of the higher-temperature shape is smaller than the cylinder diameter of the lower-temperature shape.

In the aforesaid embodiment, the cylinder diameter of the lower-temperature shape is constant cylinder diameter D1 from one opening end to the other opening end and the cylinder diameter of the higher-temperature shape is constant cylinder diameter D2. However, when the cylinder diameter D2 of higher-temperature is set larger than the cylinder diameter D1 of lower-temperature, sufficient enlargement of the diameter sometimes cannot be obtained at the opening end (i.e., the opening end is narrowed in approximately tapered shape when the diameter of the intermediate portion is enlarged to the cylinder diameter D2).

Such problem can be solved by setting the opening of both ends larger than the intermediate portion in the lower-temperature shape (first shape).

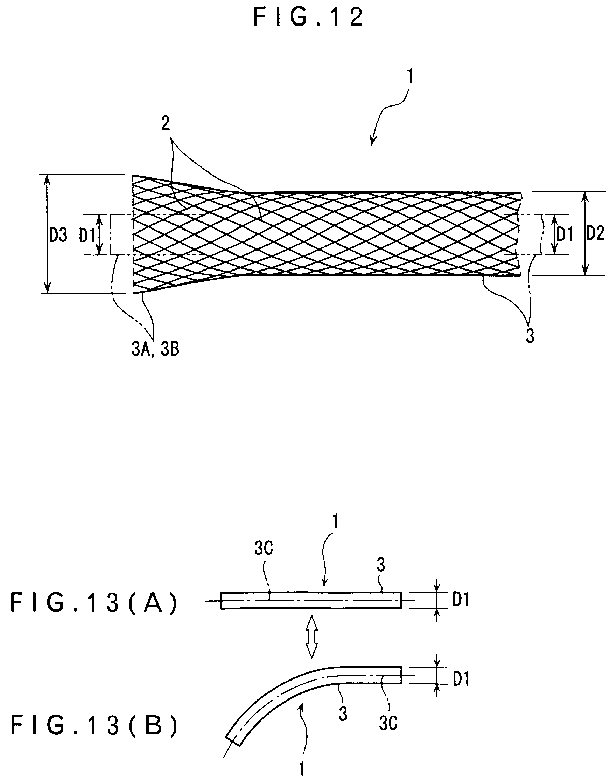

In the other embodiment shown in Fig. 12, the stent 1 is configured approximately the same as the embodiment of Fig. 1, where the hollow cylinder body 3 entirely has the cylinder diameter Dl in the lower-temperature shape as the first shape. However, in the higher-temperature shape as the second shape, though the intermediate portion thereof has cylinder diameter D2, the opening portions 3A and 3B of both ends are enlarged to be cylinder diameter D3.

According to the present embodiment, in addition to the same effect as described in the Fig. 1 to Fig. 10, the fluidity of humor is unlikely to be influenced in indwelling into the intracorporeal lacuna since the opening end is not narrowed in widening the diameter.

In the aforesaid embodiment, both of the lower-temperature shape (first shape) and the higher-temperature shape (second shape) of the stent 1 is linear tube with variable tube the tube diameter. However, the change between the respective shapes is not restricted to the change in tube diameter, and the changing shape is not restricted to the linear tube.

For instance, either the first shape in lower-temperature or the second shape in higher-temperature may be curved tube or bent L-shape.

In the still other embodiment shown in Fig. 13, the hollow cylindrical body 3 of the stent 1 has the first shape in lower-temperature with tube diameter D1 and straight axial line 3C as shown in Fig 13(A). On the other hand, as shown in Fig. 13(B), though the tube diameter of the second shape in higher-temperature remains D1, the axial line 3C is curved in arc shape. Such stent 1 is in a linear shape in lower-temperature as shown in Fig. 13(A), which changes in a curved shape in higher-temperature as shown in Fig. 13(B).

According to such change, the stent 1 can be more appropriately applied to a curved intracorporeal lacuna, thereby allowing the humor or fluid substance passing through the intracorporeal lacuna to flow smoothly.

Incidentally, the tube diameter may be set variable accompanying the above-described deformation of the axial line.

When the tube diameter is changed, the diameter is not restricted to be uniformly enlarged on the entirety of the stent 1. In other words, diameter enlarging ratio may be set large on one end and diameter enlarging ratio may be set small on the other end, so that the second shape may be tapered-tube shape even with the first shape of tube shape of entirely the same diameter.

Though the fixing step for fixing the crossed portion of the filament body 2 at both ends of the hollow cylindrical body 3 is conducted after the lower-temperature shape memorizing process in the aforesaid embodiment, other arrangement is possible.

Specifically, the fixing step may be conducted prior to lower-temperature shape memorizing process or after higher-temperature shape memorizing process.

## Claims

1. A stent comprising a hollow cylindrical body made of a plurality of filament body of Ti-Ni alloy having excessive Ni, the hollow cylindrical body memorizing first shape during lower-temperature shape memorizing process and second shape during higher-temperature shape memorizing process and being defonnable between the first shape and the second shape in accordance with temperature change.

2. The stent according to Claim 1, wherein the lower-temperature shape memorizing process is a solution thermal treatment and the higher-temperature shape memorizing process is a constraint aging thermal treatment.

3. The stent according to Claim 1 or 2, wherein the first shape has smaller cylinder diameter than the second shape.

4. The stent according to Claim 3, wherein crossed portion of the filament body is fixed only at both ends of the hollow cylindrical body.

5. The stent according to Claim 3 or 4, wherein the first shape of the hollow cylindrical body has smaller diameter than the second shape and the second shape has larger diameter of both opening ends than an intermediate portion thereof.

6. The stent according to any one of Claims 1 to 5, wherein one or more than one of the plurality of filament body is made of gold or tantalum.

7. The stent according to any one of Claims 1 to 6, wherein the Ti-Ni alloy contains Co or Cu.

8. A manufacturing method of a stent, comprising the steps of: braiding a plurality of filament body of Ti-Ni alloy of excessive Ni to form a hollow cylindrical body defonnable into mutually different first shape and second shape;
memorizing lower-temperature shape for memorizing the first shape to the hollow cylindrical body as a lower-temperature shape; and
memorizing higher-temperature shape for memorizing the second shape to the hollow cylindrical body as a higher-temperature shape.

9. The manufacturing method of a stent according to Claim 8, wherein a solution thermal treatment is conducted during the lower-temperature shape memorizing step while keeping the hollow cylindrical body in the first shape, and wherein an aging thermal treatment is conducted during the higher-temperature shape memorizing step while binding the hollow cylindrical body in the second shape.

10. An indwelling method of a stent according to any one of Claims 1 to 8, comprising the steps of:
providing a stent having first shape of lower-temperature with larger cylinder diameter than cylindrical diameter of second shape of higher-temperature;
accommodating the stent into a sheath after making it thinner than the cylinder diameter of the first shape;
inserting a distal end of the sheath adjacent to indwelling position of intracorporeal lacuna; and
inserting a rod into the sheath and pushing out the stent in the sheath to indwell the stent in the intracorporeal lacuna.
